# EUROPEAN PATENT SPECIFICATION

(11) **EP 4 448 474 B1**
(45) Date of publication and mention of the grant of the patent: **12.08.2026**
(21) Application number: 22839642.0
(22) Date of filing: 15.12.2022
(51) Int. Cl.: C07C 29/42, C07C 33/048

(54) **PROCESS FOR ETHYNYLATING SPECIFIC ALPHA,BETA-UNSATURATED KETONES**
VERFAHREN ZUR ETHINYLIERUNG VON SPEZIFISCHEN ALPHA,BETA-UNGESÄTTIGTEN KETONEN
PROCÉDÉ D'ALCYNYLATION DE CÉTONES INSATURÉES SPÉCIFIQUES ALPHA,BETA

(30) Priority: 17.12.2021 EP 21215524
(43) Date of publication of application: 23.10.2024
(73) Proprietor: DSM IP Assets B.V., 6221 BE Maastricht (NL)
(72) Inventor: BONRATH, Werner, 4303 Kaiseraugst (CH); KUENZI, Rolf, 4303 Kaiseraugst (CH); NIETO-ORTEGA, Belen, 4303 Kaiseraugst (CH); MEDLOCK, Jonathan Alan, 4303 Kaiseraugst (CH)
(74) Representative: DSM IP ANH
(86) International application number: PCT/EP2022/086014
(87) International publication number: WO 2023/111119

(56) References cited:
- WO-A1-2014/078310
- US-A- 4 320 236
- K. R. MARTIN ET AL: "Ethynylation of Ketones Using Dilithium Acetylide", THE JOURNAL OF ORGANIC CHEMISTRY, VOL. 33, N°2, 1 February 1968 (1968-02-01), pages 778 - 780, XP055170759, Retrieved from the Internet <URL:http://pubs.acs.org/doi/pdf/10.1021/jo01266a060> [retrieved on 20150219], DOI: 10.1021/jo01266a060

## Description

The present invention relates to an improved process for ethynylating specific α,β-unsaturated ketones for producing tertiary acetylenic alcohols.

The process for ethynylating α,β-unsaturated ketones for producing tertiary acetylenic alcohols is well known and has been disclosed and described in patent applications and publications. For example, in US4320236 or Chimia, 40(9), 1986 p.323 - 330 such an ethynylation is described in detail. In Martin et al., The Journal of Organic Chemistry, 33, 1968, p. 778-780 the ethynylation of ketones using dilithium acetylide is described.

The present invention relates to an improved process to produce a compound of formula (I) 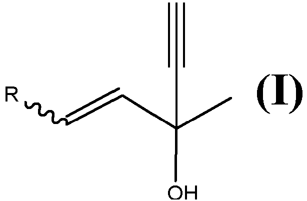 wherein R is H, an aliphatic or aromatic hydrocarbon moiety and the wavy bond means that the carbon-carbon double bond to which it is attached to, can be in the (E) or (Z) configuration.

Such compounds can be used as such, or additionally they can be used intermediates in the synthesis of other industrially relevant compounds, (for example vitamin A derivatives and carotenoids)

Due to the importance of the compounds of formula (I), there is always a need to provide improved syntheses.

As stated above there are a few ways to produce compounds of formula (I).

According to the literature (such as US4320236 or Chimia, 40(9), 1986 p.323 - 330), the specific ethynylation can be summarized as done in the following scheme:

### Step (I)

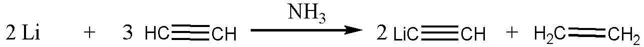

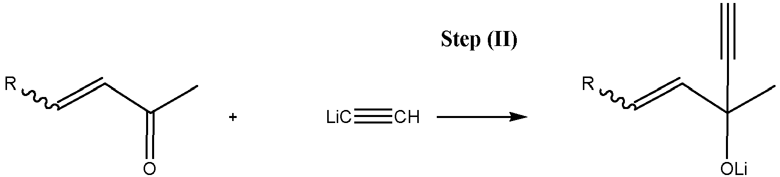

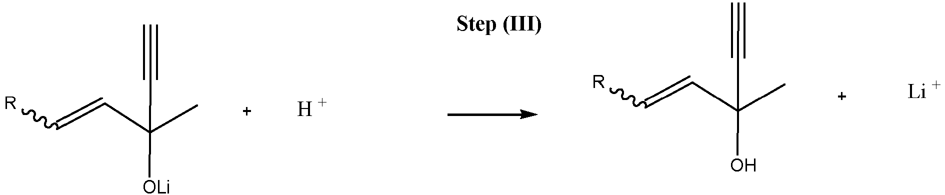

wherein R is as defined above and in more detail below.

Proposed by the literature mentioned above, step (I) consists of the following two steps (step (Ia) and step (Ib)):

### Step (Ia)

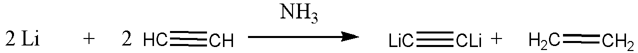

### Step (Ib)

LiC≡CLi + HC≡CH → 2 LiC≡CH.

One of the main disadvantages and downsides of the process described in the prior art is the control of the process.

The processes of the prior art use the starting materials in a large excess to achieve good yield, resulting in a large amount of waste. Particularly, in the case of an excess of unsaturated ketones such as those of formula (II), oligomers and polymers can be produced which are very difficult to remove and result in a significant yield loss.

Furthermore, the correct moment of the addition of the starting material in the various steps (step (I), step (II) and step (III)) is also challenging. If the starting material is added too early or too late that can lead to unwanted side products, which then have to be removed in an time-consuming and extensive process.

In chemical reactions using the correct stoichiometry of reagents is important, the ideal situation is to only exactly the right amount of the reagent to completely consume the starting material. If an excess of one component (starting material or reagent) is used, this generally results in lower yields and higher costs due to the value of the unreacted excess. An excess of reagents can result in a more hazardous reaction mixture and so require more safety controls. In addition, the excess of this reagent has to be removed from the product, potentially requiring further expensive purification steps. Furthermore, an excess of one component can react to produce by-products, which require removal from the product. Specifically, compounds of formula (II) are known to be toxic and also susceptible to polymerization. Polymers cause significant difficulties and extra costs in their removal in purification steps. Thus, the amounts of reagents used in the reaction should be minimized and their addition to a reaction stopped as soon as the other reaction components are completely consumed.

Therefore, the goal of the present invention was to find a way and a process to produce specific tertiary acetylenic alcohols, which reduces the amount of unwanted side products.

Surprisingly, it was found when the reaction progress was monitored by using Raman spectroscopy it was possible to find the ideal point in time to add the various starting material and also to stop the reactions at the ideal moments.

Raman spectroscopy (named after Indian physicist C. V. Raman) is a non-destructive vibrational spectroscopic technique which provides exhaustive information about molecular interactions, polymorphism, crystallinity, and chemical structure in general.

Raman spectroscopy is based upon the called Raman effect, in which the incident light from a high intensity laser light source is scattered from a sample at different wavelength as laser source, which depend on the chemical structure of the sample.

Therefore, a Raman spectrum features the intensity and the wavenumber position of the scattered light which can be correlated with specific molecular bonds, allowing the identification of unknown samples, or monitoring the reaction path of the substances.

Some functional groups are more Raman active than others and will produce more intense bands. For example, the C=O bond characteristic of aldehydes is not strongly Raman-active. Alternatively, triple bonds such as C=C are highly active.

The Raman spectroscopy can be carried by using any commonly available instrument. There are multiple producers and suppliers for Raman equipment, for example Kaiser Optical Systems, Bruker and Mettler Toledo. These instruments can be fitted with a variety of probes and optics for the analysis of liquids, solids and gases. A very suitable way to carry out the Raman spectroscopy in a chemical reaction is by using an immersion probe, which is put into the reaction mixture, inside the vessel.

The process according to present invention is carried out the following way:
In step (la), the lithium metal is added to NH₃, and then the ethyne (HCCH) is added forming the lithium carbide.

### Step (Ia)

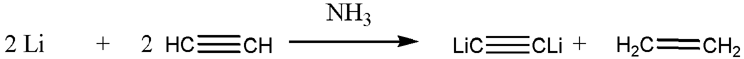

Then when more ethyne (HCCH) is added (step (Ib)), the lithium carbide is converted into lithium acetylide.

### Step (Ib)

LiC≡CLi + HC≡CH → 2 LiC≡CH

The important feature is to determine the exact point where the lithium carbide is completely converted to the lithium acetylide. At this point the addition of the ketone of formula (II) is added 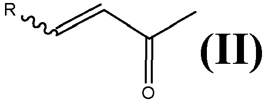 wherein R is as defined above and in more detail below.

If the addition of the ketone of formula (II) is too early or too late then a significant amount of unwanted side products is produced. Such side products have to be removed by applying a time-consuming and extensive purification process.

Furthermore, also step (II) can be controlled better by following the reaction using Raman spectroscopy: 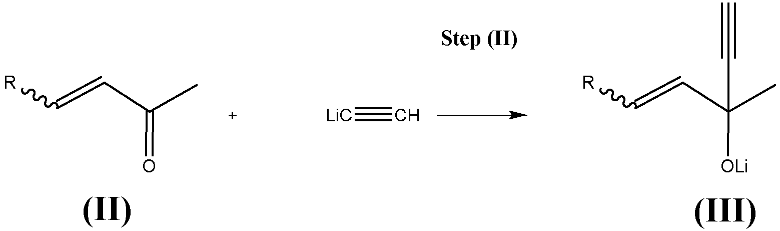 wherein R is as defined above and in more detail below.

As described above, the correct point to add the ketone of formula (II) can be determined by Raman spectroscopy. Additionally, Raman spectroscopy can be used to determine when all lithium acetylide is consumed to identify the exact moment when to stop the addition of the ketone of formula (II).

In the final stage (step III)) 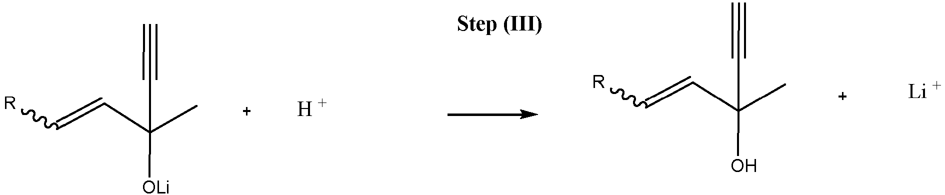
wherein R is as defined above and in more detail below,
the hydrolysis is carried out to obtain the compound of formula (I) in excellent yield. The hydrolysis of the compound of general formula (III) can be affected in a manner known per se, such as but not limited to use of Bronsted acids such as sulfuric acid, acetic acid, water, ammonium chloride.

As stated above the amount of unwanted side products and waste is reduced significantly in the overall process.

Therefore, the present invention relates to a process (P) for the production of compounds of formula (I) 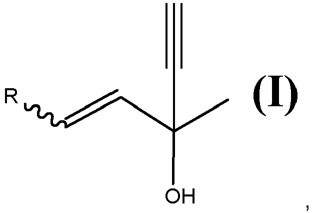 wherein
R is H; a linear, branched or cyclic C₁-C₃₀ alkyl group, which can comprise ring systems and, which can be substituted with oxygen atoms; or a linear, branched or cyclic C₂ - C₃₀ alkylene moiety, which can comprise ring systems and, which can be substituted with oxygen atoms, wherein
a first step lithium is added to NH₃ and then
ethyne (HCCH) is added to the reaction mixture and
in a second step (step (II)
a compound of formula (II) 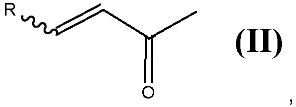
wherein
R and the wavy bond have the same meaning as defined for the compound of formula (I), and
then in a third step (step (III)) the obtained product is hydrolyzed,
characterized in that the step (I) and step (II) and optionally step (III) are controlled by monitoring the reaction progress by using Raman spectroscopy.

In a preferred embodiment of the present invention, R is H; a linear, branched or cyclic C₁-C₁₅ alkyl group, which can comprise ring systems and, which can be substituted with oxygen atoms; or a linear, branched or cyclic C₁-C₁₅ alkenyl group, which can comprise ring systems and, which can be substituted with oxygen atoms.

In a more preferred embodiment of the present invention, R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 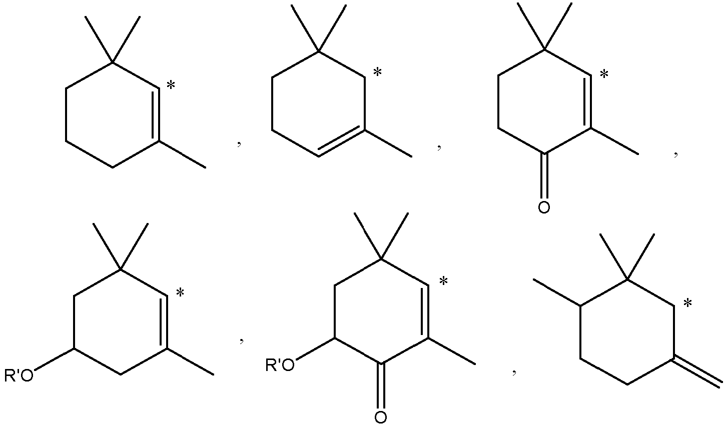 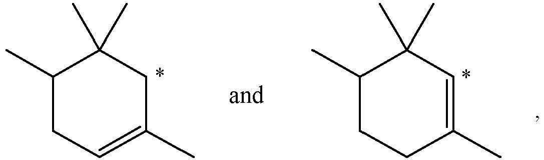
wherein the "*" shows the C bonding to the compound of formula (I) and compound of formula (II) and
R' is H or a C₁-C₄ alkyl group or -(CO)C₁-C₄alkyl or -C(COCH₃)(CH₃)₂.

In an even more preferred embodiment of the present invention, R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 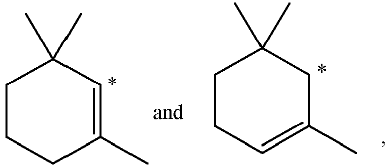 wherein the "*" shows the C bonding to the formula (I) and (II).

In a most preferred embodiment of the present invention the compound of formula (I') 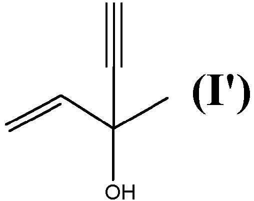 is used.

Therefore, the present invention also relates to a process (P'), which is process (P), wherein R is H; a linear, branched or cyclic C₁-C₁₅ alkyl group, which can comprise ring systems and, which can be substituted with oxygen atoms; or a linear, branched or cyclic C₁-C₁₅ alkenyl group, which can comprise ring systems and, which can be substituted with oxygen atoms.

Therefore, the present invention also relates to a process (P"), which is process (P), wherein R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 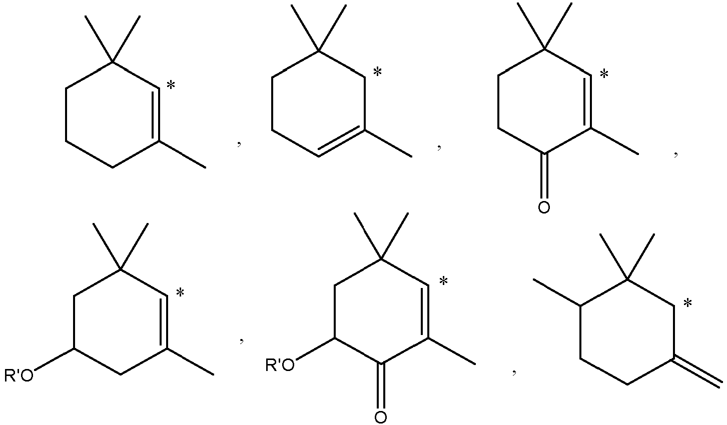 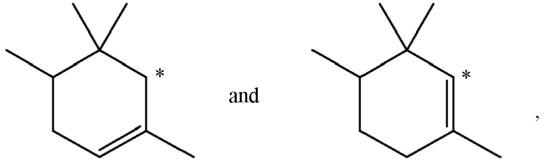
wherein the "*" shows the C bonding to the formula (I) and (II) and
R' is H or a C₁-C₄ alkyl group or -(CO)C₁-C₄alkyl or -C(COCH₃)(CH₃)₂.

Therefore, the present invention also relates to a process (P‴), which is process (P), wherein
R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 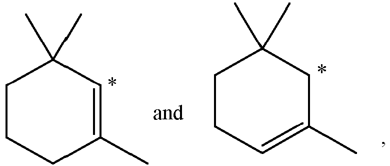
wherein the "*" shows the C bonding to the formula (I) and (II).

Therefore, the present invention also relates to a process (Pʺʺ), which is process (P), wherein the compound of formula (I') 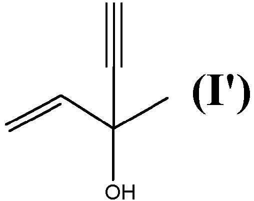 is used.

The Raman spectroscopy is used to determine when to add or when to stop adding the various reaction compounds.

As stated above the by introducing the wavy bond (in formula (I) and formula (II)), it is meant, that the compound of formula (I) can be the compound of formula (la) or the compound of formula (Ib) 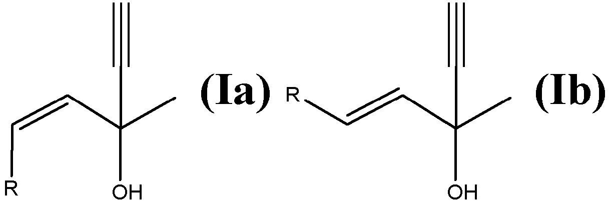
wherein R is as defined above
as well a mixture of the compound of formula (la) and the compound of formula (Ib) in any ratio (R has the same meaning as defined above).

The same applies for the compound of formula (II) and of formula (III).

Any commonly known and commercially available Raman spectroscopy device can be used. Preferably an immersion probe attached to a Raman analyzer or Raman spectrometer. Such Raman devices are commercially available from a variety of producers and suppliers. Raman immersion probes can be integrated easily into the process equipment.

All values given in the present patent application are measured with a Raman spectrometer from Kaiser Optical Systems (Kaiser Raman Rxn2 analyzer). This spectrometer is equipped with a CCD detector, which allows a full Raman spectrum to be recorded in a few seconds. The 785 nm laser was used at 50 mW.

For the experiments described in the present patent, spectra were recorded every minute, with a resolution of better than 2 cm⁻¹. An exposure time of 10 seconds was used during the complete reaction time. No fluorescence effects were observed.

As stated above the exact point of the addition of the various reagents during the process is determined by Raman spectroscopy.

The reaction conditions for the ethynylation are similar to those disclosed in US4320236.

Step (I) (both steps Ia and Ib) is usually carried out at a temperature range of from -90°C to -10 °C. The lithium metal is usually added with stirring.

Therefore, the present invention also relates to a process (P1), which is process (P), (P'), (P"), (P‴) or (Pʺʺ), wherein step (I) is carried out at a temperature range of from -90°C to - 10 °C.

In step (la)

### Step (Ia)

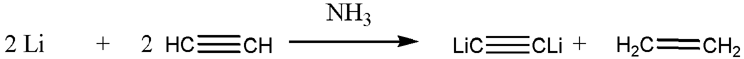

the Raman spectroscopy is used to determine when the lithium has first been added to the ammonia and then to monitor the formation of the lithium carbide.

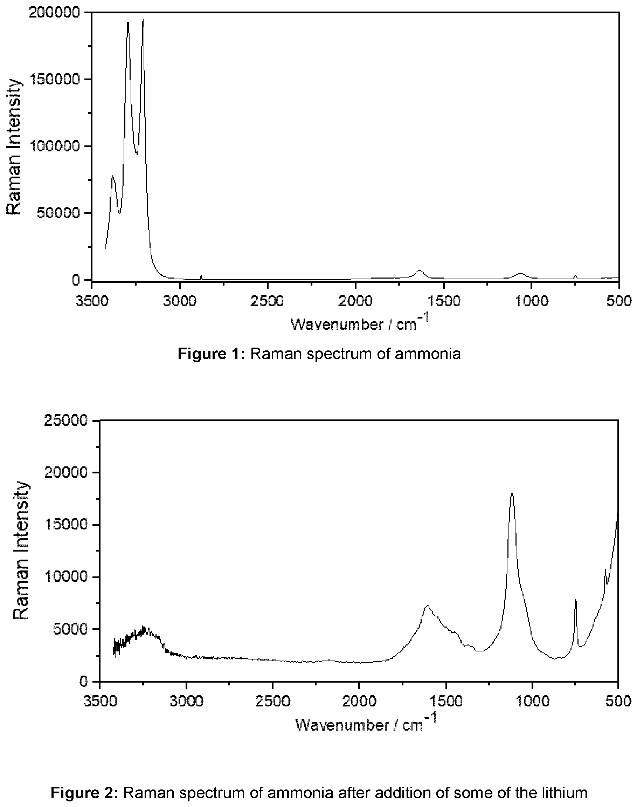

At the beginning of the process according to the present invention (no lithium added to the NH₃ yet) three peaks can be seen between 3000 cm⁻¹ to 3500 cm⁻¹ (see Figure 1) (the N-H stretching band).

When adding the lithium to the NH₃ the three peaks between 3000 cm⁻¹ to 3500 cm⁻¹ disappear in this region (see Figure 2) and a shift is observed in the N-H bending bands.

After addition of the lithium is complete, the dosing of acetylene is started.

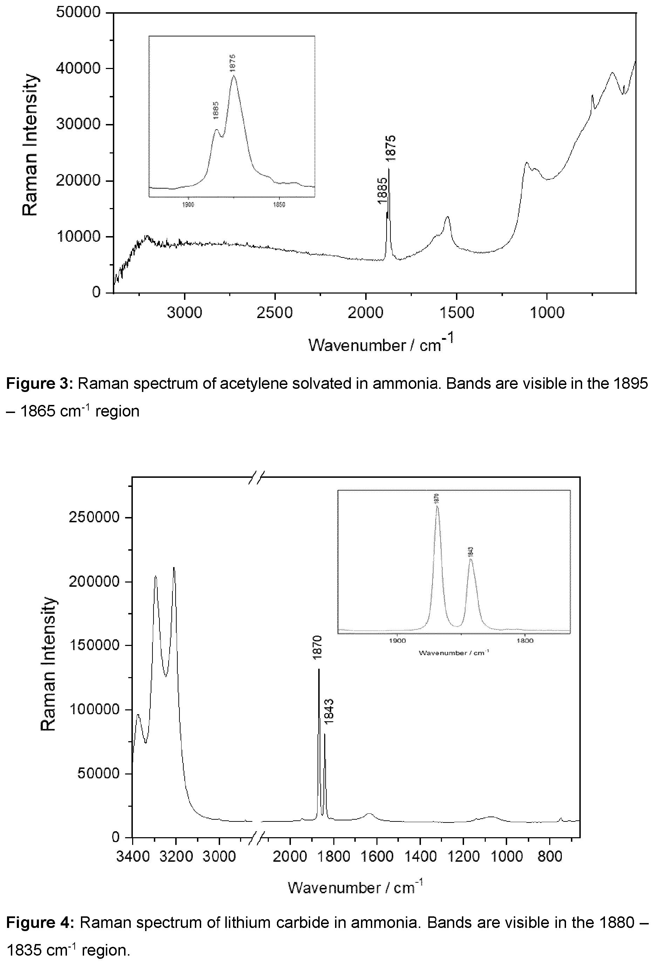

The dosing of acetylene can be followed by Raman spectroscopy. Two bands are recorded in the region of 1895 to 1865 cm⁻¹, which can be attributed to an acetylene solvated species (Figure 3).

Continuing to add acetylene, two Raman bands of the solvate species disappear and two bands, in the region of 1880 to 1835 cm⁻¹, are detected (Figure 4). In this moment, the addition of acetylene can be stopped, the lithium carbide is formed.

Raman spectroscopy indicates when the formation of lithium carbide is completed, and the addition of acetylene is stopped.

Therefore, the present invention also relates to process (P2), which is process (P), (P'), (P"), (P‴), (Pʺʺ) or (P1), wherein the dosing of acetylene is stopped when the new peaks (in the region of 1880 to 1835 cm⁻¹) appear.

At this point, at least one inert solvent is added to the reaction mixture. Suitable inert solvents for the process according to the present invention are ethers and aromatic hydrocarbon compounds, such as i.e., diethyl ether, di-n-propyl ether, diisopropyl ether, dioxane, tetrahydrofuran, 2-methyl tetrahydrofuran, benzene and toluene.

Therefore, the present invention also relates to process (P3), which is process (P), (P'), (P"), (P‴), (Pʺʺ), (P1) or (P2), wherein at least one inert solvent is added to the reaction mixture when two peaks in the region of 1880-1835 cm⁻¹ are observed.

Therefore, the present invention also relates to process (P3'), which is process (P3), wherein the at least one inert solvent is chosen from the group consisting of ethers and aromatic hydrocarbon compounds.

Therefore, the present invention also relates to process (P3"), which is process (P3), wherein the at least one inert solvent is chosen from the group consisting of diethyl ether, di-n-propyl ether, diisopropyl ether, dioxane, tetrahydrofuran, 2-methyl tetrahydrofuran, benzene and toluene.

In step (Ib), after the addition of the at least one solvent more acetylene is added to the reaction mixture:

### Step (Ib)

LiC≡CLi + HC≡CH → 2 LiC≡CH.

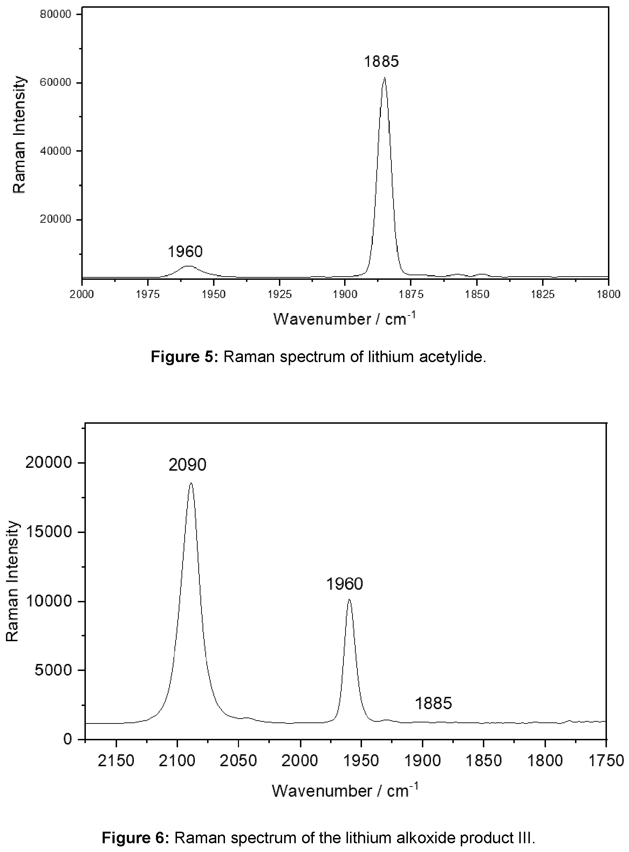

After the solvent exchange (from NH₃ to the at least one inert solvent) and further addition of acetylene, the bands of the lithium carbide decrease (two bands 1880-1835 cm⁻¹) and the lithium acetylide can be detected. At this point total conversion to lithium acetylide is reached and it can be observed by Raman (band at approximately 1885 cm⁻¹) (see Figure 5).

Therefore, the present invention also relates to process (P4), which is process (P), (P'), (P"), (P‴), (Pʺʺ), (P1), (P2), (P3), (P3') or (P3"), wherein the dosing of acetylene is stopped when two peaks in the region of 1880-1835 cm⁻¹ have disappeared and the peak in the region of 1885 cm⁻¹ appears.

At this point (Step II), the compound of formula (II) 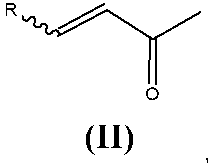 wherein
R has the same meanings as defined above,
is added to the reaction mixture and the compound of formula (III) 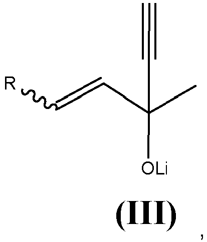
wherein
R has the same meanings as defined above,
is formed.

Reaction scheme of step (II): 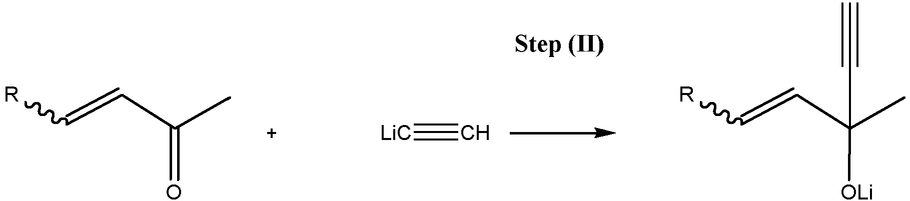 R has the same meanings as defined above.

The compound of formula (II) is added to the reaction mixture until the Raman band approximately at 1885 cm⁻¹ has disappeared. At this point, the addition of the compound of formula (II) is stopped (see Figure 6).

Raman spectroscopy indicates the point of total consumption of the lithium acetylide and the formation of the compound of formula (III) (band at approximately 2090 cm⁻¹).

Therefore, the present invention also relates to process (P5), which is process (P), (P'), (P"), (P‴), (Pʺʺ), (P1), (P2), (P3), (P3'), (P3") or (P4), wherein the dosing of the compound of formula (II) is stopped when the band at approximately 1885 cm⁻¹ has disappeared.

The temperature at step (II) is usually between -70°C and 0°C.

Therefore, the present invention also relates to process (P6), which is process (P), (P'), (P"), (P‴), (Pʺʺ), (P1), (P2), (P3), (P3'), (P3"), (P4) or (P5), wherein step (II) is carried out at a temperature between -70°C and 0°C.

The final step (step (III)) 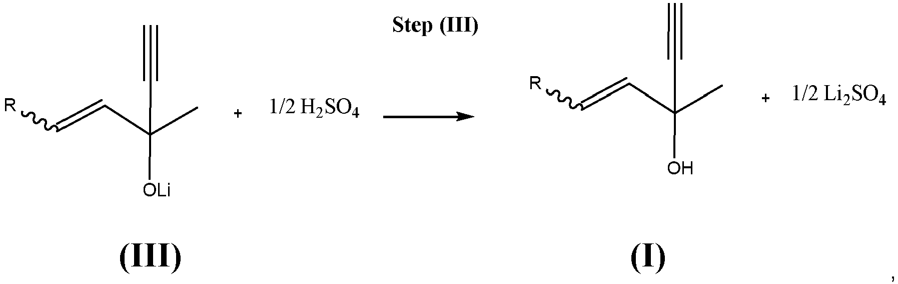
wherein R has the same meanings as defined above,
is less critical in regard of producing unwanted side products, so therefore it is optional if this step is followed by Raman spectroscopy.

The step (III), which is the hydrolysis step, is usually carried out temperature of from -70°C and 0°C, preferably -40°C to -5°C.

Therefore, the present invention also relates to process (P7), which is process (P), (P'), (P"), (P‴), (Pʺʺ), (P1), (P2), (P3), (P3'), (P3"), (P4), (P5) or (P6), wherein step (III) is carried out at a temperature between -70°C and 0°C.

Therefore, the present invention also relates to process (P7'), which is process (P), (P'), (P"), (P‴), (Pʺʺ), (P1), (P2), (P3), (P3'), (P3") (P4), (P5) or (P6), wherein step (III) is carried out at a temperature between -40°C to -5°C.

The hydrolysis in step (III) is carried out by using at least one Bronsted acid, such as: sulfuric acid, acetic acid, water, ammonium chloride.

Therefore, the present invention also relates to process (P8), which is process (P), (P'), (P"), (P‴), (Pʺʺ), (P1), (P2), (P3), (P3'), (P3"), (P4), (P5), (P6), (P7) or (P7'), wherein step (III) the at least compound is chosen from the group consisting of sulfuric acid, acetic acid, water and ammonium chloride.

Therefore, the present invention also relates to process (P9), which is process (P), (P'), (P"), (P‴), (Pʺʺ), (P1), (P2), (P3), (P3'), (P3"), (P4), (P5), (P6), (P7), (P7') or (P8), wherein step (III) is carried out at a temperature between -70°C and 0°C.

Therefore, the present invention also relates to process (P9'), which is process (P), (P'), (P"), (P‴), (Pʺʺ), (P1), (P2), (P3), (P3'), (P3"), (P4), (P5), (P6), (P7), (P7') or (P8), wherein step (III) is carried out at a temperature between -40°C to -5°C.

At the end of step (III), the compound of formula (I) is obtained and isolated and purified by using commonly known methods.
Figure 1: Raman spectrum of ammonia
Figure 2: Raman spectrum of ammonia after addition of some of the lithium
Figure 3: Raman spectrum of acetylene solvated in ammonia
Figure 4: Raman spectrum of lithium carbide
Figure 5: Raman spectrum of lithium acetylide
Figure 6: Raman spectrum of the lithium alkoxide product III.
Figure 7: Raman spectra of the reactions Example 1 and Comparison Example 1 (C1)

The following examples illustrate the invention further without limiting it. All percentages and parts, which are given, are related to the weight and the temperatures are given in °C, and the pressures are absolute pressures when not otherwise stated.

### EXAMPLES

### Example 1

Ammonia gas is condensed into a cooled (-50 to -30 °C) 2L jacketed vessel fitted with a Raman probe under argon until the vessel contains approximately 500 ml of liquid ammonia. Lithium metal (10.5 g) is slowly added with stirring (Figure 1 -> Figure 2). Acetylene gas is added to the reaction mixture at a rate of approximately 1 L/min (Figure 3). The addition of acetylene is stopped when the Raman spectrum indicated formation of the lithium carbide (Figure 4).

The reaction temperature is increased to between -10 and +10 °C and diethyl ether (approximately 625 ml) is added. The reaction mixture is cooled to -15 to -5 °C and acetylene gas is added at a rate of approximately 1 L/min. When complete formation of the lithium acetylide is observed (Figure 5), a solution of methyl vinyl ketone (120 g in 120 ml of diethyl ether) is prepared and is added to the lithium acetylide solution at approximately 3.3 ml/min. The reaction is monitored by Raman spectroscopy and as soon as the consumption of the lithium acetylide is complete (Figure 6), the addition is stopped and the unused methyl vinyl ketone solution is disposed of (approx. 171 g of MVK solution added). The reaction mixture is stirred and then added over approximately 20 minutes to a cooled solution of sulfuric acid (30%, approximately 400 ml).

The ether layer is separated, dried over sodium sulfate and most of the ether is removed at normal pressure to give the crude 3-methylpent-1-en-4-yn-3-ol as a yellow oil (248.5g, 53.3 weight% content, 97.9% yield based on MVK and 91.3% yield based on lithium).

### Comparative Example 1 (C1) (not using a Raman Spectroscopy to monitor the reaction progress)

The procedure of Example 1 was repeated, without the use of Raman spectroscopy using the same amounts of lithium metal and methyl vinyl ketone solution. The point at which the lithium acetylide was fully consumed was estimated to be reached after the addition of approximately 193g of the MVK solution (approx. 1.59 mol).

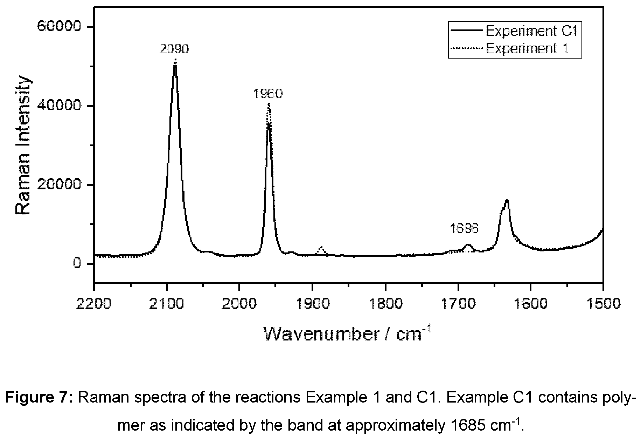

After work-up, 255.8g of crude 3-methylpent-1-en-4-yn-3-ol was obtained as a yellow oil (51.5 weight% content, 86.6% yield based on MVK). In addition, a polymer was formed (see Figure 7) that made the removal of the mixture from the reactor and purification significantly harder.

### Comparative Example 2 (C2) (not using a Raman Spectroscopy to monitor the reaction progress)

The procedure of Example 1 was repeated, without the use of Raman spectroscopy using the same amounts of lithium metal and methyl vinyl ketone solution. The point at which the lithium acetylide was fully consumed was estimated to be reached after the addition of approximately 146g of the MVK solution (approx. 1.20 mol).

After work-up, 209.8g of crude 3-methylpent-1-en-4-yn-3-ol was obtained as a yellow oil (52.9 weight% content, 96.3% yield based on MVK and 76.5% yield based on lithium).

## Claims

1. Process for the production of compounds of formula (I) 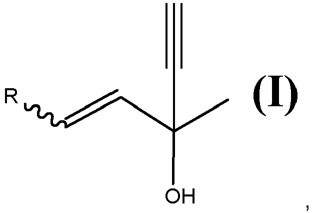 wherein
R is H; a linear, branched or cyclic C₁-C₃₀ alkyl group, which can comprise ring systems and, which can be substituted with oxygen atoms; or a linear, branched or cyclic C₂ - C₃₀ alkylene moiety, which can comprise ring systems and, which can be substituted with oxygen atoms, wherein
a first step lithium is added to NH₃ and then
ethyne (HCCH) is added to the reaction mixture and
in a second step (step (II)
a compound of formula (II) 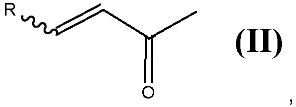
wherein
R and the wavy bond have the same meaning as defined for the compound of formula (I), and
then in a third step (step (III)) the obtained product is hydrolyzed,
**characterized in that** the steps (I) and step (II) and optionally step (III) are controlled by monitoring the reaction progress by using Raman spectroscopy.

2. Process according to claim 1, wherein R is H; a linear, branched or cyclic C₁-C₁₅ alkyl group, which can comprise ring systems and, which can be substituted with oxygen atoms; or a linear, branched or cyclic C₁-C₁₅ alkenyl group, which can comprise ring systems and, which can be substituted with oxygen atoms.

3. Process according to claim 1, wherein R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 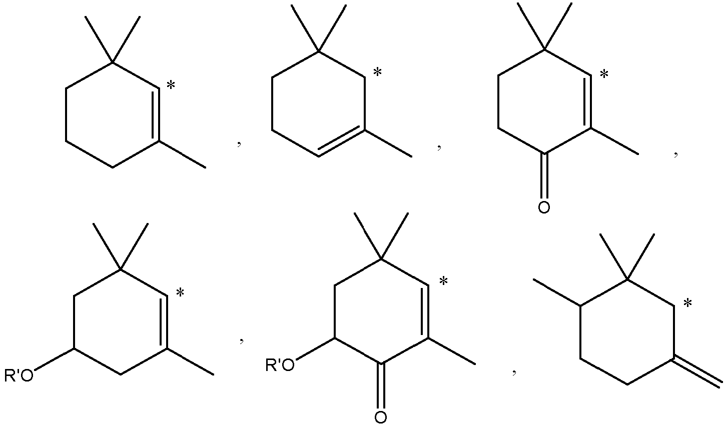 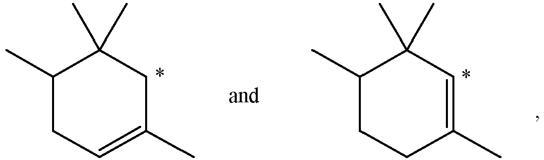
wherein the "*" shows the C bonding to the formula (I) and (II) and
R' is H or a C₁-C₄ alkyl group or -(CO)C₁-C₄alkyl or -C(COCH₃)(CH₃)₂.

4. Process according to claim 1, wherein R is H; a linear or branched C₁-C₁₀ alkyl group; a linear, branched or cyclic C₁-C₁₅ alkenyl group, which comprise one carbon-carbon double bond; or a substituted cyclohexene ring chosen from the group consisting of 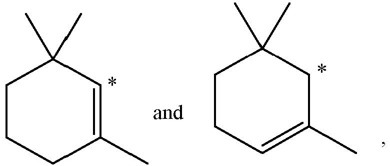 wherein the "*" shows the C bonding to the formula (I) and (II).

5. Process according to claim 1, wherein R is H.

6. Process according to any of the preceding claims, wherein step (I) is carried out at a temperature range of from -90°C to -10 °C.

7. Process according to any of the preceding claims, wherein the dosing of acetylene is stopped when new peaks in the region of 1880-1835 cm⁻¹ appear.

8. Process according to any of the preceding claims, wherein at least one inert solvent is added to the reaction mixture when two peaks in the region of 1880-1835 cm⁻¹ are observed.

9. Process according to claim 8, wherein the at least one inert solvent is chosen from the group consisting of ethers and aromatic hydrocarbon compounds.

10. Process according to claim 8, wherein the at least one inert solvent is chosen from the group consisting of diethyl ether, di-n-propyl ether, diisopropyl ether, dioxane, tetrahydrofuran, 2-methyl tetrahydrofuran, benzene and toluene.

11. Process according to any of the preceding claims, wherein the dosing of acetylene is stopped when two peaks in the region of 1880-1835 cm⁻¹ have disappeared and the peak approximately at 1885 cm⁻¹ appears.

12. Process according to any of the preceding claims, wherein the dosing of the compound of formula (II) 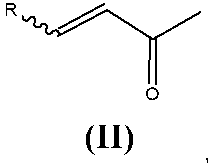 wherein
R is H; a linear, branched or cyclic C₁-C₃₀ alkyl group, which can comprise ring systems and, which can be substituted with oxygen atoms; or a linear, branched or cyclic C₂ - C₃₀ alkylene moiety, which can comprise ring systems and, which can be substituted with oxygen atoms.is stopped when the band approximately at 1885 cm⁻¹ has disappeared.

13. Process according to any of the preceding claims, wherein step (II) is carried out at a temperature between -70°C and 0°C.

14. Process according to any of the preceding claims, wherein step (III) is carried out at a temperature between -70°C and 0°C.

15. Process according to any of the preceding claims, wherein step (III) the at least compound is chosen from the group consisting of sulfuric acid, acetic acid, water and ammonium chloride.

## Patentansprüche

1. Verfahren zur Produktion von Verbindungen der Formel (I), 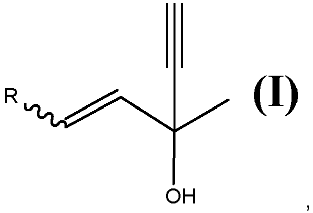 wobei
R H; eine lineare, verzweigte oder cyclische C₁-C₃₀-Alkylgruppe, die Ringsysteme umfassen kann und die mit Sauerstoffatomen substituiert sein kann; oder ein linearer, verzweigter oder cyclischer C₂-C₃₀-Alkylenanteil ist, der Ringsysteme umfassen kann und mit Sauerstoffatomen substituiert sein kann, wobei in einem ersten Schritt Lithium zu NH₃ gegeben wird, und dann
zu der Reaktionsmischung Ethin (HCCH) gegeben wird, und in einem zweiten Schritt (Schritt (II))
eine Verbindung der Formel (II) 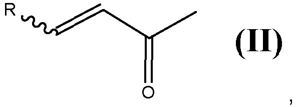
wobei
R und die wellenförmige Bindung die gleiche Bedeutung wie für die Verbindung der Formel (I) definiert haben, und dann in einem dritten Schritt (Schritt (III)) das erhaltene Produkt hydrolysiert wird,
**dadurch gekennzeichnet, dass** die Schritte (I) und Schritt (II) und gegebenenfalls Schritt (III) durch Überwachung des Reaktionsfortschritts mittels Raman-Spektroskopie gesteuert werden.

2. Verfahren nach Anspruch 1, wobei R H; eine lineare, verzweigte oder cyclische C₁-C₁₅-Alkylgruppe, die Ringsysteme umfassen kann und die mit Sauerstoffatomen substituiert sein kann; oder eine lineare, verzweigte oder cyclische C₁-C₁₅-Alkenylgruppe ist, die Ringsysteme umfassen kann und mit Sauerstoffatomen substituiert sein kann.

3. Verfahren nach Anspruch 1, wobei R H; eine lineare oder verzweigte C₁-C₁₀-Alkylgruppe; eine lineare, verzweigte oder cyclische C₁-C₁₅-Alkenylgruppe, die eine Kohlenstoff-Kohlenstoff-Doppelbindung umfasst; oder ein substituierter Cyclohexenring ist, der ausgewählt ist aus der Gruppe bestehend aus 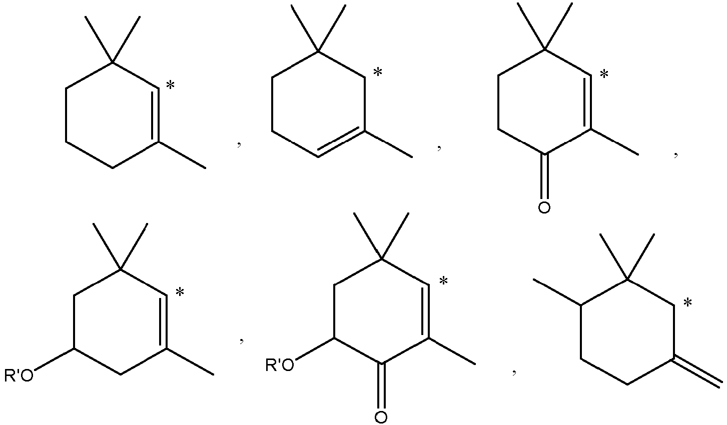 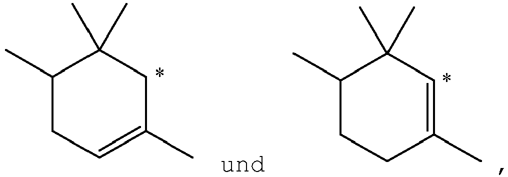
wobei das "*" das C zeigt, das an die Formel (I) und (II) gebunden ist, und
R' H oder eine C₁-C₄-Alkylgruppe oder -(CO)C₁-C₄-Alkyl oder-C(COCH₃)(CH₃)₂ ist.

4. Verfahren nach Anspruch 1, wobei R H; eine lineare oder verzweigte C₁-C₁₀-Alkylgruppe; eine lineare, verzweigte oder cyclische C₁-C₁₅-Alkenylgruppe, die eine Kohlenstoff-Kohlenstoff-Doppelbindung umfasst; oder ein substituierter Cyclohexenring ist, der ausgewählt ist aus der Gruppe bestehend aus 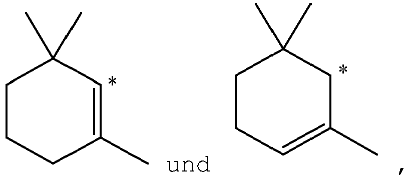 wobei das "*" das C zeigt, das an die Formel (I) und (II) gebunden ist.

5. Verfahren nach Anspruch 1, wobei R H ist.

6. Verfahren nach einem der vorhergehenden Ansprüche, wobei Schritt (I) bei einer Temperatur im Bereich von - 90 °C bis -10 °C durchgeführt wird.

7. Verfahren nach einem der vorhergehenden Ansprüche, wobei das Eindosieren von Acetylen gestoppt wird, wenn neue Peaks im Bereich von 1880-1835 cm⁻¹ erscheinen.

8. Verfahren nach einem der vorhergehenden Ansprüche, wobei der Reaktionsmischung mindestens ein inertes Lösungsmittel zugegeben wird, wenn zwei Peaks in der Region von 1880-1835 cm⁻¹ beobachtet werden.

9. Verfahren nach Anspruch 8, wobei das mindestens eine inerte Lösungsmittel ausgewählt ist aus der Gruppe bestehend aus Ethern und aromatischen Kohlenwasserstoffverbindungen.

10. Verfahren nach Anspruch 8, wobei das mindestens eine inerte Lösungsmittel ausgewählt ist aus der Gruppe bestehend aus Diethylether, Di-n-propylether, Diisopropylether, Dioxan, Tetrahydrofuran, 2-Methyltetrahydrofuran, Benzol und Toluol.

11. Verfahren nach einem der vorhergehenden Ansprüche, wobei das Eindosieren von Acetylen gestoppt wird, wenn zwei Peaks in der Region von 1880-1835 cm⁻¹ verschwunden sind und der Peak bei annähernd 1885 cm⁻¹ erscheint.

12. Verfahren nach einem der vorhergehenden Ansprüche, wobei das Eindosieren der Verbindung der Formel (II) 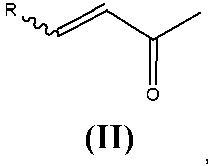 wobei
R H; eine lineare, verzweigte oder cyclische C₁-C₃₀-Alkylgruppe, die Ringsysteme umfassen kann und die mit Sauerstoffatomen substituiert sein kann; oder ein linearer, verzweigter oder cyclischer C₂-C₃₀-Alkylenanteil ist, der Ringsysteme umfassen kann und mit Sauerstoffatomen substituiert sein kann, gestoppt wird, wenn die Bande bei annähernd 1885 cm⁻¹ verschwunden ist.

13. Verfahren nach einem der vorhergehenden Ansprüche, wobei Schritt (II) bei einer Temperatur zwischen -70 °C und 0 °C durchgeführt wird.

14. Verfahren nach einem der vorhergehenden Ansprüche, wobei Schritt (III) bei einer Temperatur zwischen -70 °C und 0 °C durchgeführt wird.

15. Verfahren nach einem der vorhergehenden Ansprüche, wobei in Schritt (III) die mindestens eine Verbindung ausgewählt ist aus der Gruppe bestehend aus Schwefelsäure, Essigsäure, Wasser und Ammoniumchlorid.

## Revendications

1. Procédé de production de composés de formule (II) 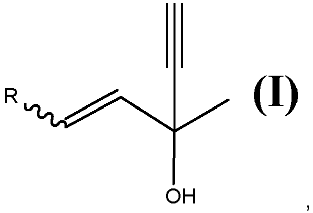 dans laquelle
R représente H ; un groupe alkyle en C₁-C₃₀ linéaire, ramifié ou cyclique, pouvant comprendre des systèmes cycliques et pouvant être substitué par des atomes d'oxygène ; ou un fragment alkylène en C₂-C₃₀ linéaire, ramifié ou cyclique, pouvant comprendre des systèmes cycliques et pouvant être substitué par des atomes d'oxygène, dans lequel
dans une première étape, du lithium est ajouté à NH₃, puis
de l'acétylène (HCCH) est ajouté au mélange réactionnel et
dans une deuxième étape (étape (II))
un composé de formule (II), 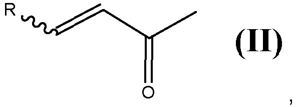
dans laquelle R et la liaison ondulée ont la même signification que pour le composé de formule (I), et
ensuite, dans une troisième étape (étape (III)), le produit obtenu est hydrolysé ;
**caractérisé en ce que** l'étape (I) et l'étape (II), et facultativement l'étape (III) sont contrôlées par le suivi de l'avancement de la réaction par spectroscopie Raman.

2. Procédé selon la revendication 1, dans lequel R représente H ; un groupe alkyle en C₁-C₁₅ linéaire, ramifié ou cyclique, pouvant comprendre des systèmes cycliques et pouvant être substitué par des atomes d'oxygène ; ou un groupe alcényle en C₁-C₁₅ linéaire, ramifié ou cyclique, pouvant comprendre des systèmes cycliques et pouvant être substitué par des atomes d'oxygène.

3. Procédé selon la revendication 1, dans lequel R représente H ; un groupe alkyle en C₁-C₁₀ linéaire ou ramifié ; un groupe alcényle en C₁-C₁₅ linéaire, ramifié ou cyclique, qui comprennent une double liaison carbone-carbone ; ou un cycle cyclohexène substitué choisi dans le groupe constitué par 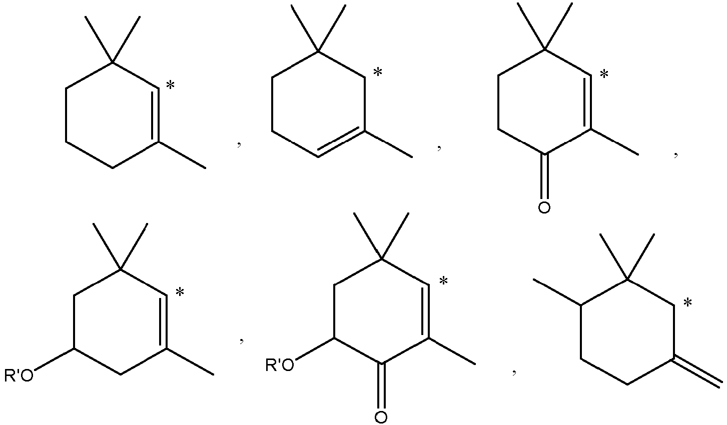 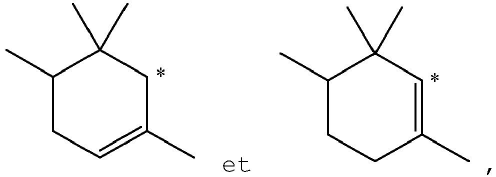
dans lequel « * » indique l'atome C lié aux formules (I) et (II) et
R' représente H ou un groupe alkyle en C₁-C₄ ou - (CO)(alkyle en C₁-C₄) ou -C(COCH₃)(CH₃)₂.

4. Procédé selon la revendication 1, dans lequel R représente H ; un groupe alkyle en C₁-C₁₀ linéaire ou ramifié ; un groupe alcényle en C₁-C₁₅ linéaire, ramifié ou cyclique, qui comprennent une double liaison carbone-carbone ; ou un cycle cyclohexène substitué choisi dans le groupe constitué par 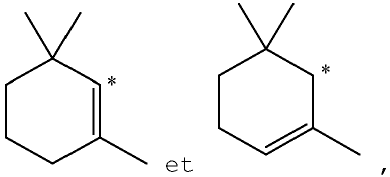 dans lequel « * » indique l'atome C lié aux formules (I) et (II).

5. Procédé selon la revendication 1, dans lequel R représente H.

6. Procédé selon l'une quelconque des revendications précédentes, dans lequel l'étape (I) est conduite à une température comprise entre -90 °C et -10 °C.

7. Procédé selon l'une quelconque des revendications précédentes, dans lequel l'ajout d'acétylène est arrêté lorsque de nouveaux pics apparaissent dans la région de 1880 à 1835 cm⁻¹.

8. Procédé selon l'une quelconque des revendications précédentes, dans lequel au moins un solvant inerte est ajouté au mélange réactionnel lorsque deux pics dans la région de 1880 à 1835 cm⁻¹ sont observés.

9. Procédé selon la revendication 8 dans lequel le ou les solvants inertes sont choisis dans le groupe constitué par les éthers et les composés hydrocarbonés aromatiques.

10. Procédé selon la revendication 8 dans lequel le ou les solvants inertes sont choisis dans le groupe constitué par l'éther diéthylique, l'éther di-n-propylique, l'éther diisopropylique, le dioxane, le tétrahydrofurane, le 2-méthyl-tétrahydrofurane, le benzène et le toluène.

11. Procédé selon l'une quelconque des revendications précédentes, dans lequel l'ajout d'acétylène est arrêté lorsque deux pics dans la région de 1880 à 1835 cm⁻¹ ont disparu et que le pic à environ 1885 cm⁻¹ apparaît.

12. Procédé selon l'une quelconque des revendications précédentes, dans lequel l'ajout du composé de formule (II) 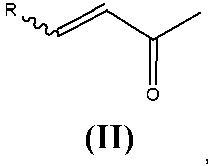 dans laquelle
R représente H ; un groupe alkyle en C₁-C₃₀ linéaire, ramifié ou cyclique, pouvant comprendre des systèmes cycliques et pouvant être substitué par des atomes d'oxygène ; ou un fragment alkylène en C₂-C₃₀ linéaire, ramifié ou cyclique, pouvant comprendre des systèmes cycliques et pouvant être substitué par des atomes d'oxygène, est arrêté lorsque la bande à environ 1885 cm⁻¹ a disparu.

13. Procédé selon l'une quelconque des revendications précédentes, dans lequel l'étape (II) est conduite à une température comprise entre -70 °C et 0 °C.

14. Procédé selon l'une quelconque des revendications précédentes, dans lequel l'étape (III) est conduite à une température comprise entre -70 °C et 0 °C.

15. Procédé selon l'une quelconque des revendications précédentes, dans lequel l'étape (III), le ou les composés utilisés sont choisis dans le groupe constitué par l'acide sulfurique, l'acide acétique, l'eau et le chlorure d'ammonium.
